**Europäisches Patentamt**

**European Patent Office**

**Office européen des brevets**

(19)

(11) **EP 1 595 941 A2**

(12) **EUROPEAN PATENT APPLICATION**

(43) Date of publication:
**16.11.2005 Bulletin 2005/46**

(51) Int Cl.$^7$: **C12M 3/00**

(21) Application number: **05251126.8**

(22) Date of filing: **25.02.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **10.05.2004 JP 2004140289**

(71) Applicant: **FUJITSU LIMITED**
**Kawasaki-shi, Kanagawa 211-8588 (JP)**

(72) Inventors:
 • **Ando, Moritoshi c/o Fujitsu Limited**
 **Kawasaki-shi Kanagawa 211-8588 (JP)**

 • **Sakai, Satoru c/o Fujitsu Limited**
 **Kawasaki-shi Kanagawa 211-8588 (JP)**
 • **Sutou, Yoshinori c/o Fujitsu Limited**
 **Kawasaki-shi Kanagawa 211-8588 (JP)**

(74) Representative: **Fenlon, Christine Lesley et al**
 **Haseltine Lake,**
 **Imperial House,**
 **15-19 Kingsway**
 **London WC2B 6UD (GB)**

(54) **Microinjectiuon device and microinjection method**

(57) A microinjection apparatus includes a substrate (12) having holes for trapping cells using suction force and injecting a substance into the cells with a needle. Height detection marks (13) are provided on the substrate (12). A visibility is calculated from an image of a height detection mark (13), and an amount of deformation of the substrate (12) is determined from the visibility. An XYZ table, on which the substrate (12) is placed, is moved based on the amount of deformation.

FIG.1

HEIGHT DETECTION MARK
13

12
Si CHIP
(Si SUBSTRATE)

EP 1 595 941 A2

**Description**

BACKGROUND OF THE INVENTION

1) Field of the Invention

The present invention relates to a technology for trapping a cell in a hole and injecting a substance into the cells with a needle.

2) Description of the Related Art

In recent years, studies on the modification of characteristics of a cell by injecting a gene into the cell as a method for the therapy of diseases due to genetic causes have been in progress. With such studies, roles of genes can be made clear and tailor made medicines that perform gene therapy suited for genetic characteristics of an individual can be prescribed.

A gene can be injected into a cell with various methods that include an electrical method (electropolation), a chemical method (lipofection), a biological method (vector method), a mechanical method (microinjection), and an optical method (laser injection).

However, the electrical method causes severe damage to the cells, the chemical method has poor efficiency, the biological method has a defect that not all the materials can be introduced in the cells. The mechanical method has received high attention as a method that is safest and exhibits high efficiency.

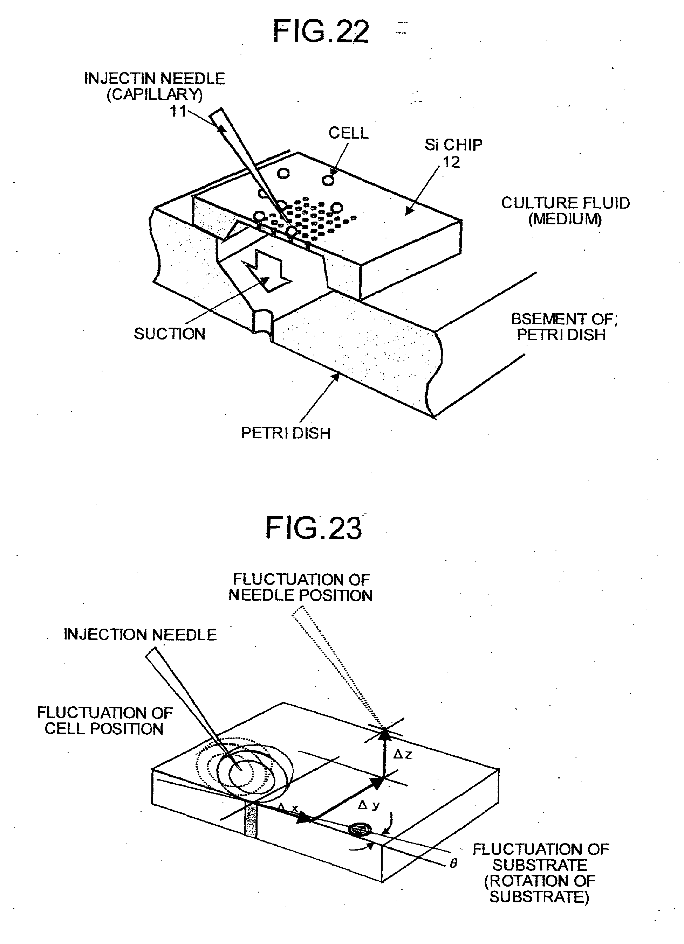

Gazette of Japanese Patent No. 2,624,719 discloses a method that performs microinjection using a capillary (injection needle) as one example of a conventional mechanical method. Fig. 22 is a schematic for explaining this method. In this method, an Si chip (also referred to as Si substrate) 12 is provided with holes, and a culture fluid (also referred to as medium) containing cells is adsorbed from below via these holes. Although not particularly depicted in subsequent explanatory diagrams, injections are performed in a state in which the holes fully filled with the culture fluid.

The holes have a diameter of the order of few micrometers ($\mu$m) and are smaller than the cells, which have a diameter of about 10 $\mu$m to 15 $\mu$m, so that the cell do not pass through the holes and remain on the Si chip 12. When the culture fluid is adsorbed, the culture fluid flows through the holes, however, the cells that flow along with the culture liquid can not pass through the holes and therefore get trapped in the holes due to the suction force. Then, a drug solution is injected into the trapped cells using an injection needle 11. A lot of cells can be processed if there are a lot of holes.

In the conventional microinjection, since the diameter of the cell is 10 $\mu$m to 15 $\mu$m, the tip of the injection needle 11 must be projected toward the central the cell at a precision of ±2 $\mu$m to ±3 $\mu$m. However, due to various reasons the position of the needle cannot be controlled so accurately.

These reasons include fluctuation of the position of the injection needle, fluctuation of shape and position of the cells, deformation of the Si chip 12, and so on. Fig. 23 is a schematic for explaining these reasons in detail.

Fluctuation of the position of the injection needle 11 is mainly due to thermal fluctuation of the shape of a needle holding mechanism (mainly in the y direction). Although not depicted in Fig. 23, an XYZ table that moves the Si chip 12 has position setting errors of several microns (in the x direction, y direction, and z direction). In addition, there are errors (of the order of few $\mu$m) in manufacturing silicon substrates, errors in alignment between the Si chip 12 and a Petri dish, and so on.

Fluctuation of the shape and position of cells include fluctuation of cell size, deviation in the centers of the holes and the cells, and so on. Since cells are living, individual cell is different in size, and generally the cells are not perfect spheres, which also make the control of the needle difficult.

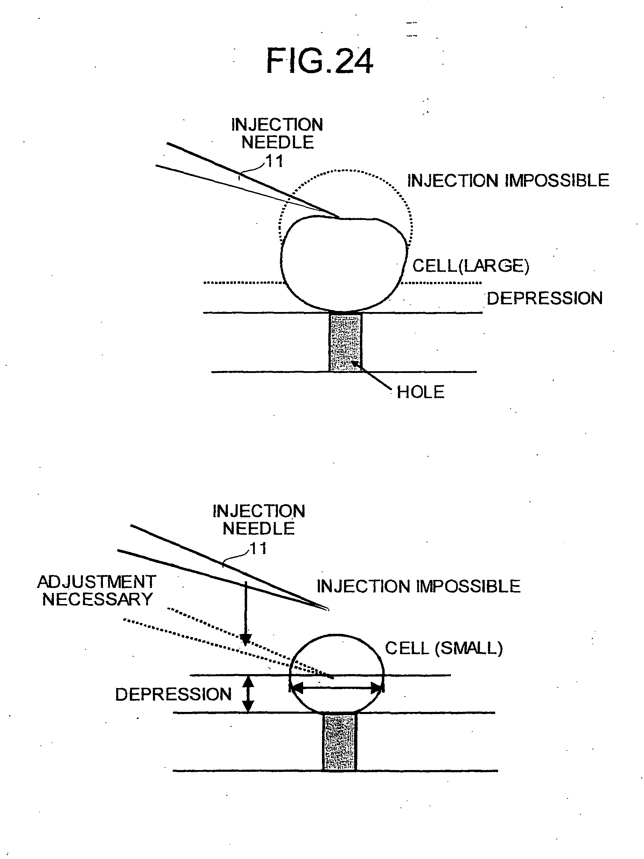

Fig. 24 is a schematic for explaining influence of variations in the height direction (z direction) of the injection needle and variations in the size of the cell size on the injection process. As shown in Fig. 24, in the case of large cells, the surface of the Si chip is depressed and the direction of the injection needle 11 is nearly parallel to the surface of the membrane of the cell, so that the tip of the injection needle 11 cannot break the cell membrane. On the other hand, in the case of small cells, the injection needle 11 does not reach the cell, so that injection can be performed.

The portion of the Si chip 12 where the holes are formed is 10 $\mu$m to 20 $\mu$m thick. Therefore, if a large number of holes are formed to perform injection to more cells at one time, the mechanical strength of this portion reduces, so that the height of this portion changes largely due to the suction from below. The amount of change in the height depends on the strength of the suction and the number of cells adsorbed.

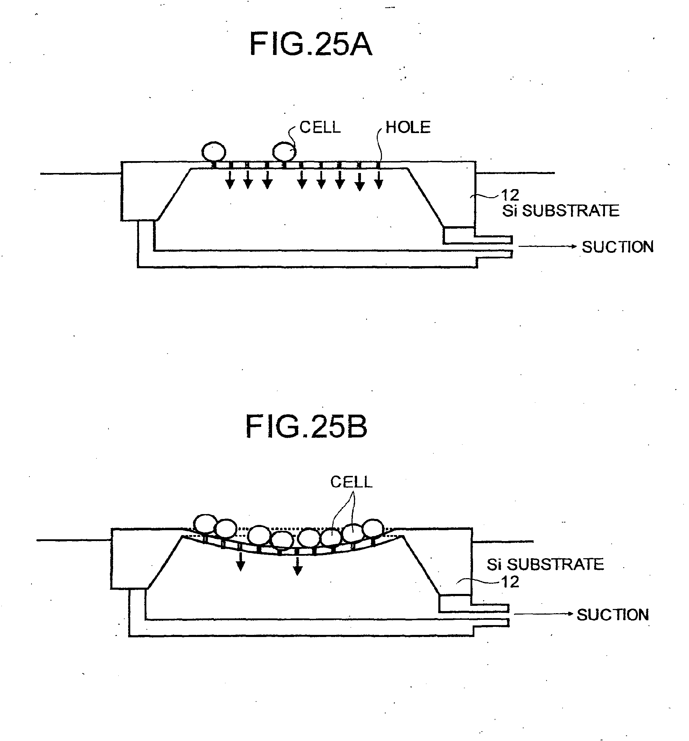

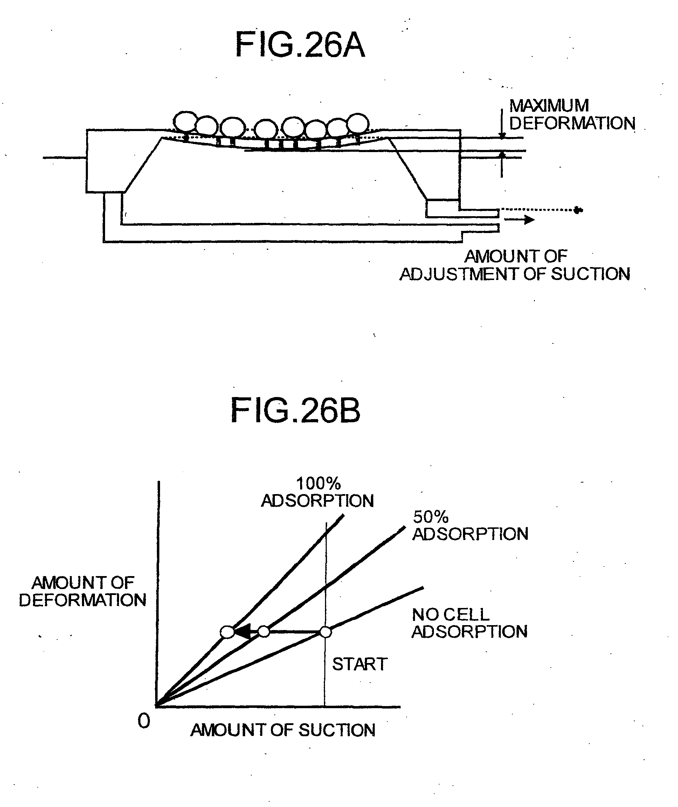

Fig. 25A is a schematic to explain the deformation of the substrate when only a few cells are trapped in the holes. When only a few cells are trapped, the culture fluid flows through the unoccupied holes, so that the substrate deforms less. On the other hand, as shown in Fig. 25B, when a large number of cells are adsorbed, the number of unoccupied holes s is small, so that when suction is continued at a constant rate, the pressure of suction portion decreases, resulting in a fluctuation of height of the substrate. The largest amount of the fluctuation is defined as a maximum deformation (see Fig. 26A).

Fig. 26B is a graph of amount of suction against amount of deformation with adsorption ratio of cells as a parameter. When no cells are adsorbed, deformation occurs to some extent. However, the amount of deformation increases with the adsorption ratio. Therefore, a method that can control the amount of deformation to a constant value is necessary.

SUMMARY OF THE INVENTION

It is an object of the present invention to solve at least the problems in the conventional technology.

According to an aspect of the present invention, a microinjection apparatus includes a substrate having a hole for trapping a cell using suction force and injecting a substance into the cell with a needle; an image acquiring unit that acquires an image of a region on the substrate, the image having characteristics that change based on a deformation of the substrate; a calculating unit that calculates an amount of deformation of the substrate based on the image acquired; and a controlling unit that controls a relative position of the needle and the substrate based on the amount of deformation.

According to another aspect of the present invention, a microinjection apparatus includes a substrate having a hole for trapping a cell using suction force and injecting a substance into the cell with a needle; a searching unit that searches a cell-free region that is a region where no cells exist on the substrate; a calculating unit that calculates a center of the cell-free region; a needle controlling unit that controls the needle so that a tip of the needle approaches the center of the cell-free region; a measuring unit that measures a position of the tip using an image of the tip while the needle is being controlled by the controlling unit; and a controlling unit that controls a relative position of the needle and the substrate based on the position of the needle measured.

According to still another aspect of the present invention, a microinjection apparatus includes a substrate having a hole for trapping a cell using suction force and injecting a substance into the cell with a needle; a measuring unit that measures a size of a cell that is trapped in the hole; and a controlling unit that controls a relative position of the needle and the substrate based on the size of the cell measured.

According to still another aspect of the present invention, a microinjection method includes trapping a cell in a hole provided in a substrate with suction force and injecting a substance into the cell with a needle; acquiring an image of a region on the substrate, the image having characteristics that change based on a deformation of the substrate; calculating an amount of deformation of the substrate based on the image acquired; and controlling a relative position of the needle and the substrate based on the amount of deformation.

According to still another aspect of the present invention, a microinjection method includes trapping a cell in a hole provided in a substrate with suction force and injecting a substance into the cell with a needle; searching a cell-free region that is a region where no cells exist on the substrate; calculating a center of the cell-free region; controlling the needle so that a tip of the needle approaches the center of the cell-free region; measuring a position of the tip using an image of the tip while the needle is being controlled; and controlling a relative position of the needle and the substrate based on the position of the needle measured.

According to still another aspect of the present invention, a microinjection method includes trapping a cell in a hole provided in a substrate with suction force and injecting a substance into the cell with a needle; measuring a size of a cell that is trapped in a hole; and controlling a relative position of the needle and the substrate based on the size of the cell.

According to still another aspect of the present invention, a microinjection apparatus includes a substrate having a hole for trapping a cell using suction force and injecting a substance into the cell with a needle; a recess around each of the holes.

The other objects, features, and advantages of the present invention are specifically set forth in or will become apparent from the following detailed description of the invention when read in conjunction with the accompanying drawings.

BRIEF DESCRIPTION OF THE DRAWINGS

Fig. 1 is a perspective of a substrate according to a first embodiment of the present invention;
Fig. 2 is a perspective of a substrate height measuring optical system of a microinjection apparatus according to the first embodiment;
Fig. 3A is a schematic of an image obtained by a CCD camera shown in Fig. 2;
Fig. 3B is an example of a height measuring signal output by the CCD camera shown in Fig. 2;
Fig. 4 is a schematic for explaining how the height of the substrate can be adjusted using the visibility;
Fig. 5 is a schematic for explaining the necessity of adjustment of height of an injection needle used for injecting a substance in the cells;
Fig. 6 is a perspective of a substrate according to a second embodiment of the present invention
Fig. 7A is a schematic for explaining the formation of a real image;
Fig. 7B is a schematic for explaining the formation of a mirror image;
Fig. 8A is an explanatory diagram for explaining another method of measuring the height of the injection needle;
Fig. 8B is a schematic of an image obtained in the situation shown in Fig. 8A;
Fig. 9 is a perspective for explaining the fluctuation in the position of the tip of the injection needle;
Fig. 10 is a schematic for explaining the problems caused due to the fluctuation of the injection needle 11;
Fig. 11A is a schematic for explaining a method of searching a cell-free region according to a third embodiment of the present invention;
Fig. 11B is a schematic for explaining coincidence of the cell-free region and the tip of the injection needle;

Fig. 12A is a schematic for explaining displacement of a cell from a hole;

Fig. 12B is a schematic for explaining relative movement of the injection needle and cell when the cell is displaced away from the hole;

Fig. 13 is a schematic for explaining an example of a method for determining the center of the cell according to a fourth embodiment of the present invention;

Fig. 14 is a schematic for explaining the effect of the size of the cell on the injection position;

Fig. 15 is a graph for explaining the relationship between the size of the cell and the amount of movement of the injection needle according to a fifth embodiment of the present invention;

Fig. 16 is a schematic for explaining the migration of a cell while the injection is performed;

Fig. 17A is a perspective of a substrate according to a sixth embodiment of the present invention;

Fig. 17B is a perspective of a cell trapped in the recess shown in Fig. 17A;

Fig. 18 is a cross-sectional view of the substrate in a situation where a cell is trapped in the recess;

Fig. 19 is a plan view of the situation shown in Fig. 18;

Fig. 20 is a plan view of a substrate according to a seventh embodiment of the present invention;

Fig. 21 is a schematic of an injection position adjustment system according to an eighth embodiment of the present invention;

Fig. 22 is a schematic for explaining a conventional microinjection method;

Fig. 23 is a schematic for explaining the drawbacks in the conventional microinjection method;

Fig. 24 is a schematic for explaining influence of variations in the height direction (z direction) of the injection needle and variations in the size of the cell size on the injection process;

Fig. 25A is a schematic for explaining the deformation of the substrate when only a few cells are trapped in the holes;

Fig. 25B is a schematic for explaining the deformation of the substrate when a lot of cells are trapped in the holes;

Fig. 26A is a schematic for explaining the maximum deformation; and

Fig. 26B is a graph of amount of suction against amount of deformation with adsorption ratio of cells as a parameter.

DETAILED DESCRIPTION

Exemplary embodiments of a microinjection apparatus and a microinjection method according to the present invention are explained in detail with reference to the accompanying drawings.

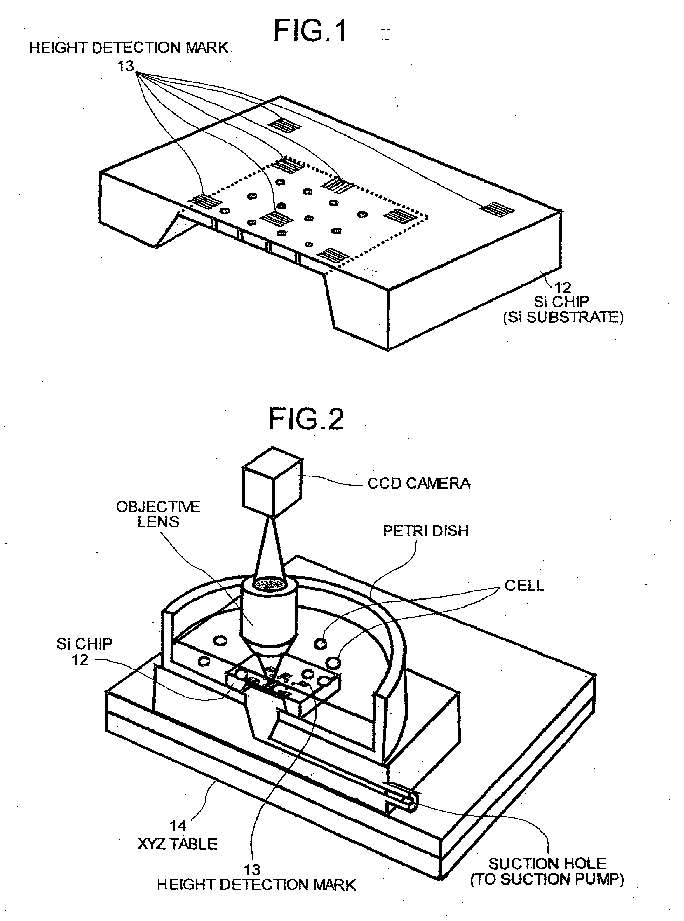

First, a microinjection apparatus that adjusts the position of the needle with respect to fluctuation of the height of the substrate is explained. Fig. 1 is a perspective of a Si chip (Si substrate) used in a microinjection apparatus according to a first embodiment. An Si chip (Si substrate) 12 is provided with a plurality of height detection marks 13 and these height detection marks 13 are used for adjusting the needle position with respect to the fluctuation of the height of the substrate.

As shown in Fig. 1, each height detection mark 13 includes a pattern made of a plurality of fine lines. These lines are parallel to the surface of the Si chip. The height detection marks 13 are formed at various positions. For example, the height detection marks 13 are formed in the center and the periphery of the region where holes are formed, and even in regions where no holes are formed.

The microinjection apparatus according to the first embodiment can measure the posture of the Si chip and flexure of hole forming region by measuring height of each of the patterns.

Fig. 2 is a perspective of a substrate height measuring optical system of the microinjection apparatus according to the first embodiment. The substrate height measuring optical system includes a water-immersed objective lens that enlarges each of the patterns on the surface of the Si chip and a CCD camera that observes each of the patterns. The Si chip 12 is adhered to a Petri dish provided with a perforated portion.

A suction hole, for sucking air through the perforated portion of the Petri dish, is formed in a board that holds the Petri dish. The suction hole is connected to a suction pump (not shown) through a tube and the suction pump sucks a culture solution (fluid). The suction rate of the suction pump can be set as desired. The board that holds the Petri dish is placed on an XYZ table 14.

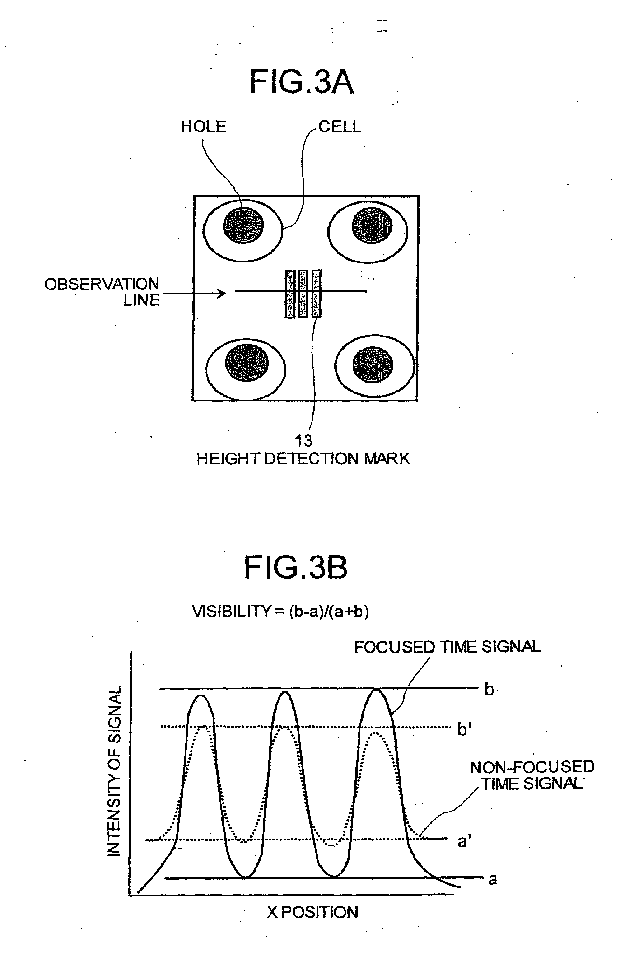

Fig. 3A is a schematic of an image obtained by the CCD camera. The CCD camera is adjusted in such a manner that the center of the pattern is in the center of the view of the CCD camera. (The observation line is directed so as to align the direction of scanning pixels of the CCD camera. In this direction, measurement with less measurement errors is possible.)

Fig. 3B is an example of a height measuring signal output by the CCD camera. When the focal point of the CCD camera lies on the pattern, a signal having a large amplitude and high visibility is output. The visibility can be calculated as Visibility = $(b-a)/(a+b)$. The visibility decreases when the focus is offset. Accordingly, by calculating the visibility, the relationship between the height of the substrate and the objective lens can be measured and an amount of depression of the substrate can be calculated.

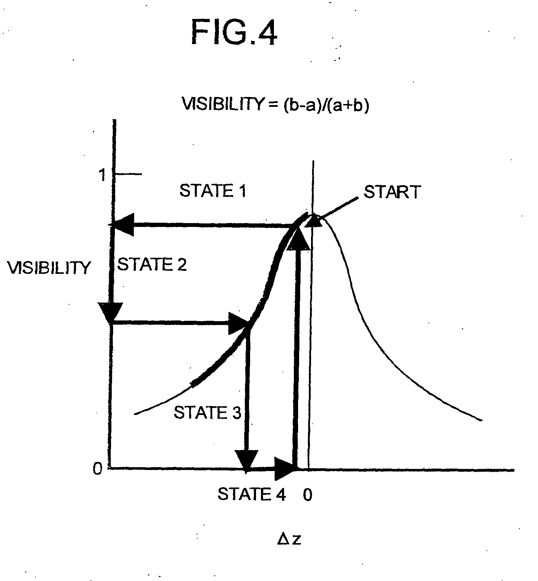

Fig. 4 is a schematic for explaining how the height of the substrate can be adjusted using the visibility. The horizontal axis indicates the height of surface of the Si chip 12 and focused focal point height is taken 0.

On the other hand, the vertical axis indicates visibility.

At the time of start, the substrate is in only slightly depressed state (state 1) because very few cells fit in the holes, and therefore, the visibility if high. As more and more cells fit in the holes, the visibility decreases (state 2), because, the amount of deformation increase (state 3). To compensate for the deformation, the XYZ table 14 is moved (or the objective lens is moved) in the Z direction to obtain higher visibility.

By repeating this operation at constant intervals, the amount of depression can be maintained at a constant value. By removing extra cells after adsorption of the cells, this state is stabilized. The relationship between the visibility and the amount of depression is acquired beforehand and the relationship between pressure and flexure is measured beforehand.

As described above, in the first embodiment, the visibility is calculated by using the height detection mark 13 provided on the surface of the Si chip 12 and observing an image of the height detection mark 13, an amount of depression of the Si chip 12 is calculated using the relationship between the calculated visibility and the height of the Si chip 12, and the XYZ table 14 is moved based on the amount of depression. Accordingly, the depression of the Si chip 12 can be compensated for with good precision. Outer peripheries of the hole in the Si chip 12 for trapping the cells can also be used as height detection marks instead of the above-mentioned patterns.

A microinjection apparatus according to a second embodiment is configured so as to adjust the height of the injection needle 11 along with the movement in the horizontal direction of the XYZ table 14. First, the necessity of adjusting the height of the injection needle 11 is explained.

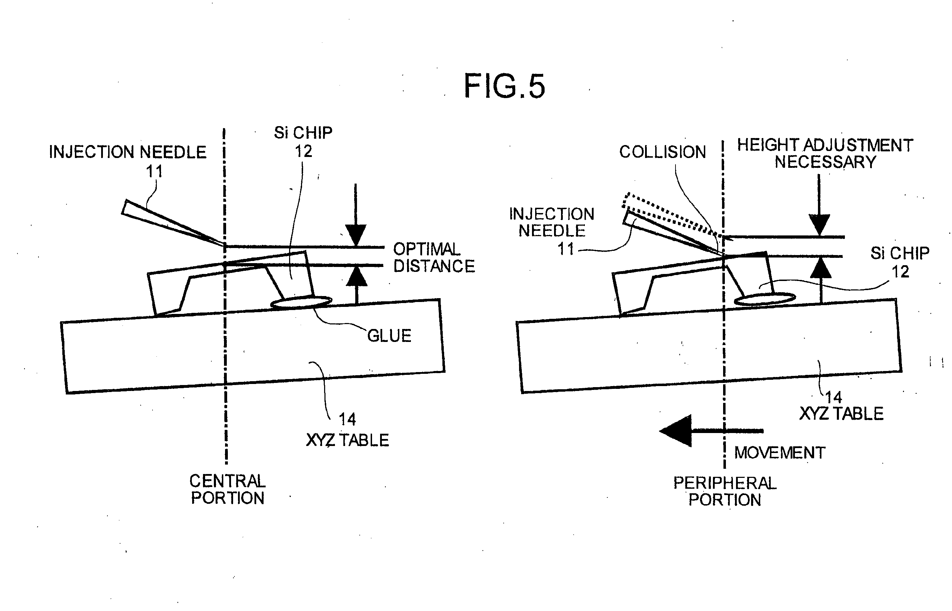

Fig. 5 is a schematic for explaining the necessity of adjustment of the height of the injection needle 11. As shown in Fig. 5, the XYZ table 14 is generally not perfectly horizontal. Since the injection needle 11 projects toward the center of the cell, the distance between the tip of the injection needle 11 and the surface of the Si chip is only about 5 μm. Therefore, when the XYZ table 14 moves in the horizontal direction, injection to the cell that is trapped on the Si chip 12 becomes impossible. Further, in some cases the injection needle 11 may collide with the Si chip 12 thereby causing damage.

When there are a large number of holes in the Si chip, the holes occupy a wider area on the Si chip, resulting in an increase in fluctuation of the height of the chip and an increase in the amount of flexure. As a result, the movement of the XYZ table 14 may bring about a situation where the injection needle 11 collides with the Si chip 12 thereby causing damage.

Accordingly, the microinjection apparatus according to the second embodiment measures a distance (height) between the needle tip and the surface of the Si chip and provides a control so as to maintain a predetermined constant distance between them. This arrangement makes it possible to project the injection needle 11 toward the center of the cell and also prevent damage due to collision of the injection needle 11 with the Si chip 12.

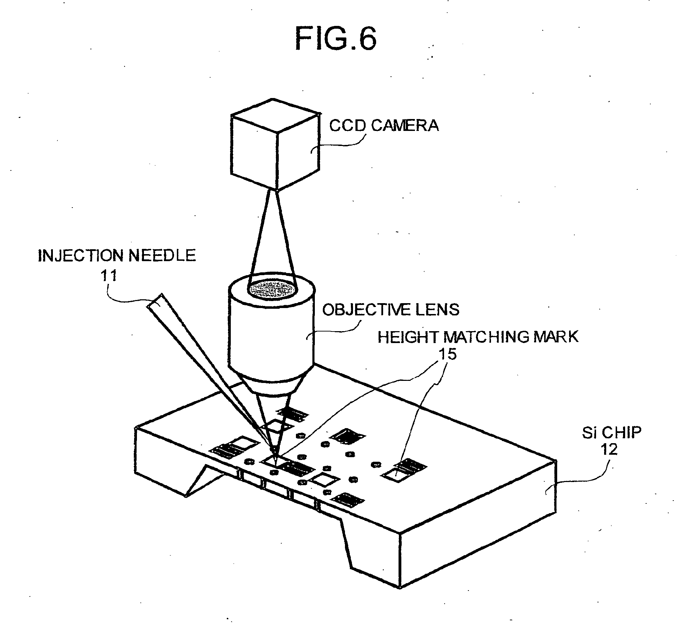

Fig. 6 is a perspective for explaining the method of measuring the distance between the Si chip surface and the needle tip. As shown in Fig. 6, a plurality of height matching marks 15 are provided on the surface of the Si chip 12. The height matching marks 15 are flat, have a predetermined area (10 μm$^2$ to 20 μm$^2$), and reflect light. Well-polished silicon surfaces can be used as the height matching marks 15. Moreover, metal surfaces can be used as the height matching marks 15. However, if the metal surfaces used, it is preferable that the metal surfaces be provided with a protective coating of $SiO_2$ so that the metal does not directly come in contact with the culture solution.

The microinjection apparatus according to the second embodiment is configured such that the tip of the injection needle 11 is positioned near the center of the height matching mark 15 and a real image of the real tip and a mirror image of the tip seen in the height matching mark 15 are observed. An objective lens and a CCD camera are used for the observation.

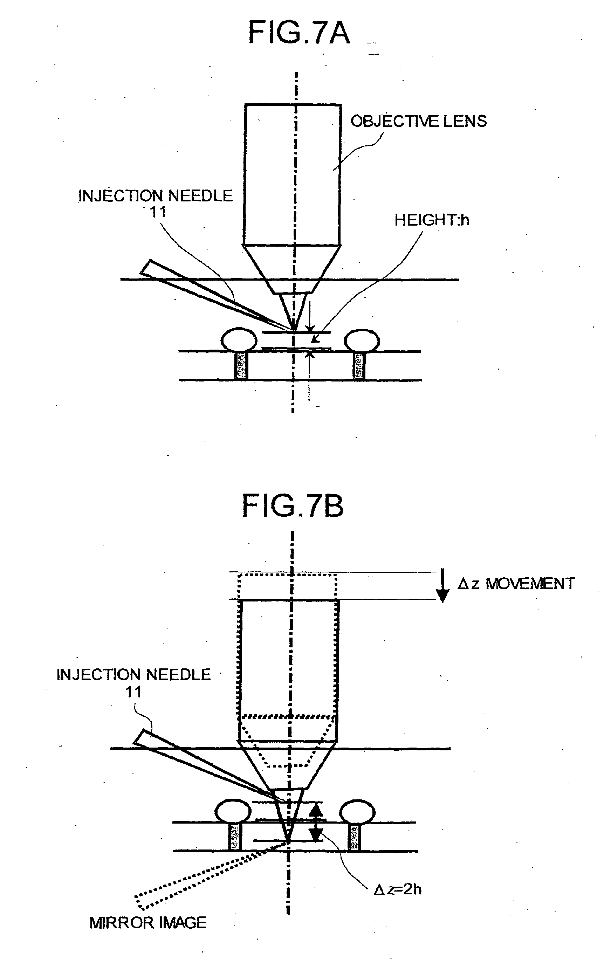

Fig. 7A is a schematic for explaining the formation of the real image and Fig. 7-2 is a cross-sectional view for explaining the formation of the mirror image. The microinjection apparatus according to the second embodiment determines shifts in position Δz in the height direction of the real image and mirror image and make the values 1/2 to measure the height of the needle.

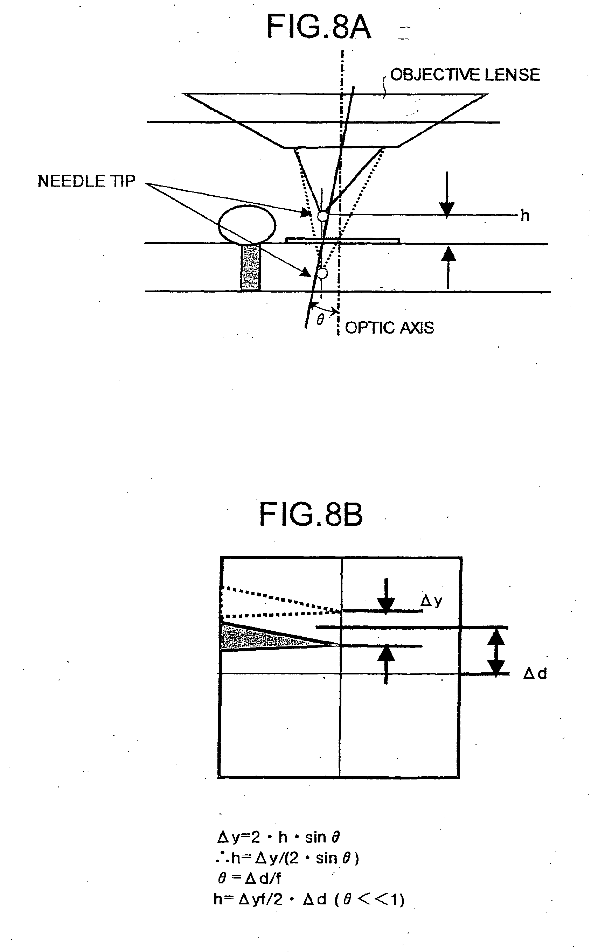

Fig. 8A is an explanatory diagram for explaining another method for measuring the height of the injection needle. In this method, the injection needle is slightly moved away horizontally from the optic axis of the objective lens. Fig. 8B is a schematic of an image obtained in the situation shown in Fig. 8A.

As shown in Fig. 8A, the objective lens of a microscope forms an image such that when an angle (θ) from the center of the lens is different, an image is formed at a different position, so that the mirror image and the real image are formed slightly different positions in the same plane.

Now, assuming that a distance between the mirror image and the real image is measured to be Δy, a needle height h can be calculated from the angle θ as follows:

$$h = \Delta y/(2\text{-}\sin\theta).$$

Here, assuming the distance between the centers of the two images and the center of the observation to be Δd, the angle θ can be calculated from:

$$\theta = \Delta d/f$$

where f is a focal length of the objective lens. The focal length of the objective lens can be calculated from:

$$h = \Delta yf/2 \cdot \Delta d$$

where $\theta \ll 1$.

As described above, according to the second embodiment, the real image and the mirror image of the injection needle are measured using the height matching marks 15 provided on the surface of the Si chip 12, and the height from the injection needle 11 is measured based on the shifts of position of the real image and the mirror image in the direction of height, so that the distance between the injection needle 11 and the Si chip 12 can be maintained at a predetermined value by moving the XYZ table 14 up and down based on the measured height.

When the XYZ table 14 has moved in the horizontal direction, the up and down movement of the XYZ table 14 is calculated using the direction of movement, distance of movement and inclination of the XYZ table 14, and the height of the XYZ table 14 is controlled so as to correct the calculated up and down movement of the XYZ table, resulting in that the injection needle 11 and the surface of the Si chip can be always maintained at a constant distance.

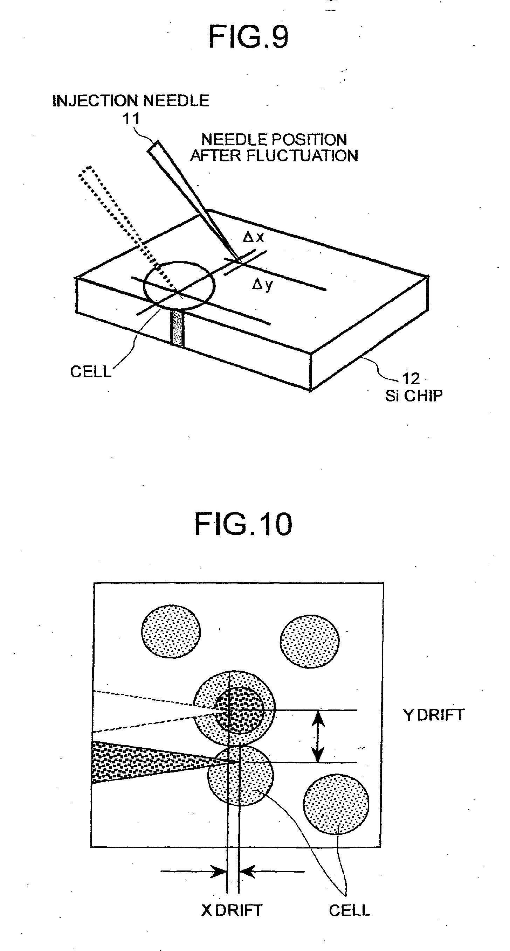

A microinjection apparatus according to a third embodiment of the present invention if configured so as to adjust the position of the needle based on the fluctuation in position of the injection needle tip. First, the fluctuation in position of the injection needle tip is explained. Fig. 9 is a perspective for explaining the fluctuation in the position of the tip of the injection needle.

As shown in Fig. 9, the injection needle 11 may fluctuate in a horizontal plane in the x- and y-directions and make the injection impossible. The injection needle 11 can fluctuate due to deformation of a needle holding mechanism or deformation of the needle itself due to a change in surrounding temperature. Due to structural peculiarities of the needle, it fluctuates more in the y-direction than in the x-direction.

Fig. 10 is a schematic for explaining the problems caused due to the fluctuation of the injection needle 11. Not all the cells are absorbed in the hole, i.e., some cells may exist in a portion other than the holes. If a cell exists below the injection needle 11 while the injection needle 11 fluctuates, an image having a poor contrast is obtained, so that the position of the tip cannot be determined accurately.

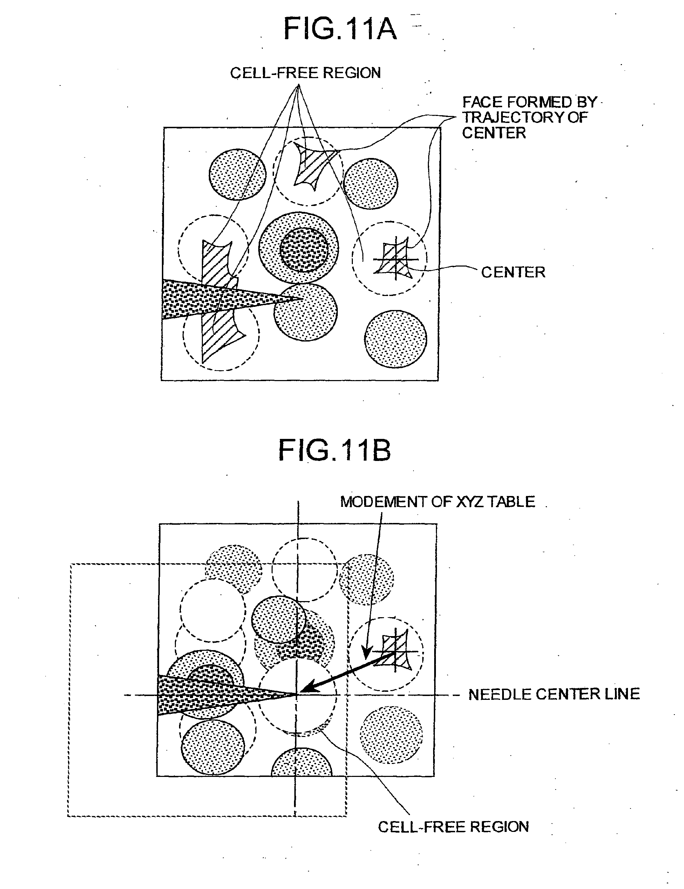

Accordingly, the microinjection apparatus according to the third embodiment searches a cell-free region out of the image including cells in order to accurately measure the position of the needle tip. Fig. 11A is a schematic for explaining a method of searching a cell-free region according to the third embodiment of the present invention.

As sown in Fig. 11A, the image is scanned using a region slightly larger than a cell (about 20 $\mu$m in diameter) as a template to search a cell-free region. In Fig. 11A, the searched cell-free region is shown as hatched. Here, the cell-free region is defined by the trajectory of the center of the search template.

Then, the center position of the largest region among the cell-free regions thus searched is obtained. In Fig. 11A, the point shown with a cross is the center position of the largest cell-free region. Then, the y-coordinate of the needle center is obtained and the XYZ table 14 is moved so that the y-coordinate of the center position shown with the cross coincide with the y-coordinate of the needle center (see Fig. 11 B). The y-coordinate is considered here, because, the needle fluctuates greater in the y direction than the x-direction.

As a result of the movement, as shown in Fig. 11 B, an image in which no cell exists under the needle can be obtained. The tip position of the needle is measured form this image. The tip position is obtained by detecting a confocal state of the image and measuring from the tip position.

As described above, in the third embodiment, a cell-free region is searched and the XYZ table 14 is moved so that the tip position of the injection needle 11 comes to a region where no cells exist, so that the tip position of the injection needle 11 can be determined accurately.

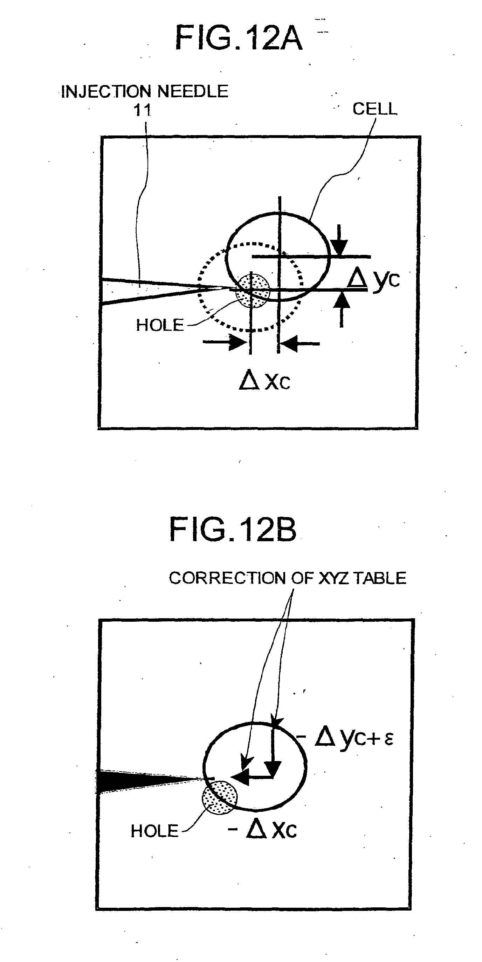

A microinjection apparatus according to a fourth embodiment of the present invention is configured so as to adjust the needle position with respect to the fluctuation of attachment position of cells. First, the fluctuation of attachment position of a cell is explained. Fig. 12A is a schematic for explaining migration of a cell.

As shown in Fig. 12A, it may occur that the center of the adsorption hole and the center of a cell do not coincide with each other since cells have various shapes. In this case, the cell is displaced by amounts $\Delta x$ and $\Delta y$ from the center of the hole. Accordingly, as shown in Fig. 12B, the microinjection apparatus according to the fourth embodiment adjusts the direction of movement of the injection needle and the position of the cell by moving the XYZ table 14.

However, when a correction is made by a fluctuation amount of $\Delta yc$ in the y-direction, a moment is applied to the cell, so that there is a possibility that the cell is out of the focus. Accordingly, the microinjection apparatus according to the fourth embodiment performs injection in a middle point between the center of the cell and the center of the hole (position shifted by $\varepsilon$ in Fig. 12B) taking into consideration adsorption force and resistance in the fluid within the cell ($\varepsilon$ is shown in Fig. 12B).

Note that the positions of the holes are known in advance, so that the displacement of the cell from the center of the hole can be calculated by determining the center of the cell. If the calculated center of the cell is not in a predetermined range of the center of the hole, no injection is performed.

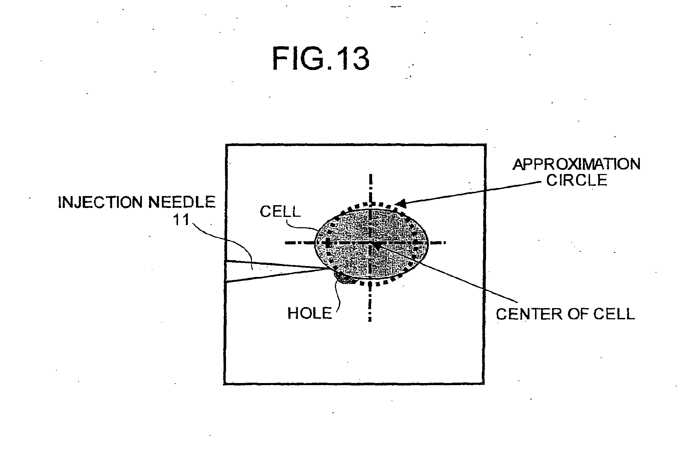

Fig. 13 is a schematic for explaining an example of a method for determining the center of the cell. Based on the fact that cells are substantially spherical, circles that resemble the contour of the cell are obtained. Then, an approximation circle that shows the smallest difference in area between the contour of the cell and the approximate circle is selected and the size and center position of this circle is defined as the position of the cell.

As described above, according to the fourth embodiment, a shift in the center of the cell is obtained by measuring the center position of the cell and obtaining a difference from the position of the hole, so that the position of the XYZ table 14 can be adjusted based on the obtained shift and injection into the cell can be performed accurately.

A microinjection apparatus according to a fifth embodiment of the present invention is configured so as to adjust the position of needle with respect to the fluctuation of the cell size. First, correction of position of injection with respect to the fluctuation of the cell size is explained.

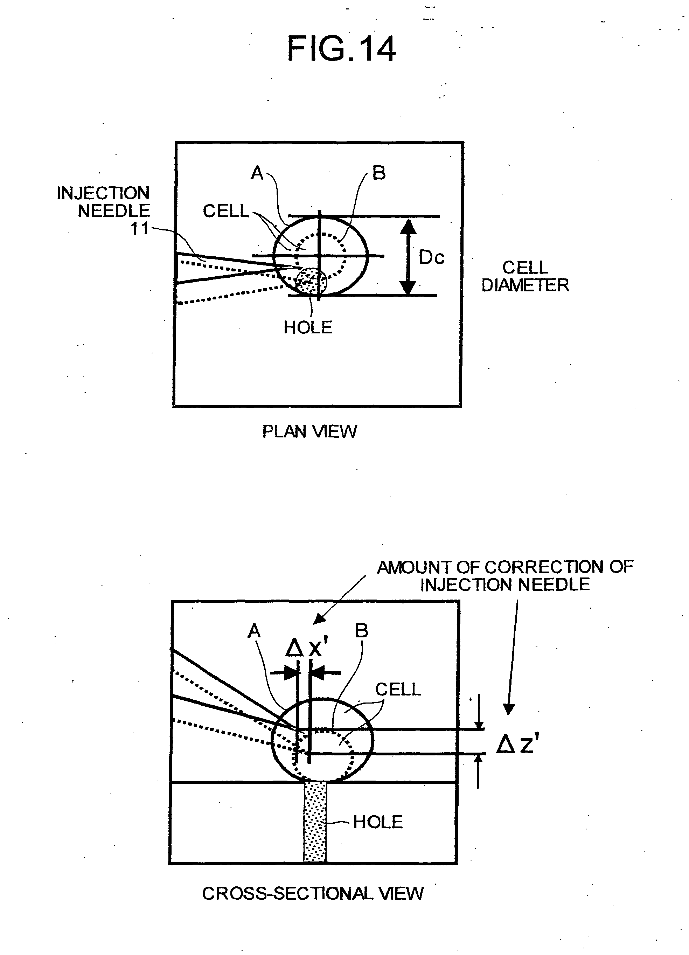

Fig. 14 is a schematic for explaining the effect of the size of the cell on the injection position. Since there are cells of various sizes, it is necessary to change the position of injection for each cell. Fig. 14 shows a plan view (above) and a cross-sectional view (below) for cases where injection is performed into a large cell A (solid line) and a small cell B (broken line), respectively.

As shown in the cross-sectional view, when injection is to be performed directed to the center of the cell, it is necessary to move the injection needle 11 to a level slightly lower ($\Delta z'$) in the case of the cell B than that in the case of the cell A. Also, in the x-direction, the injection needle 11 must be projected slightly ahead ($\Delta x'$) in the case of the small cell (cell B) as compared with the case of the cell A. Therefore, the microinjection apparatus according to the fifth embodiment measures the size of the cell and adjusts the position of the XYZ table 14 based on the size of the cell.

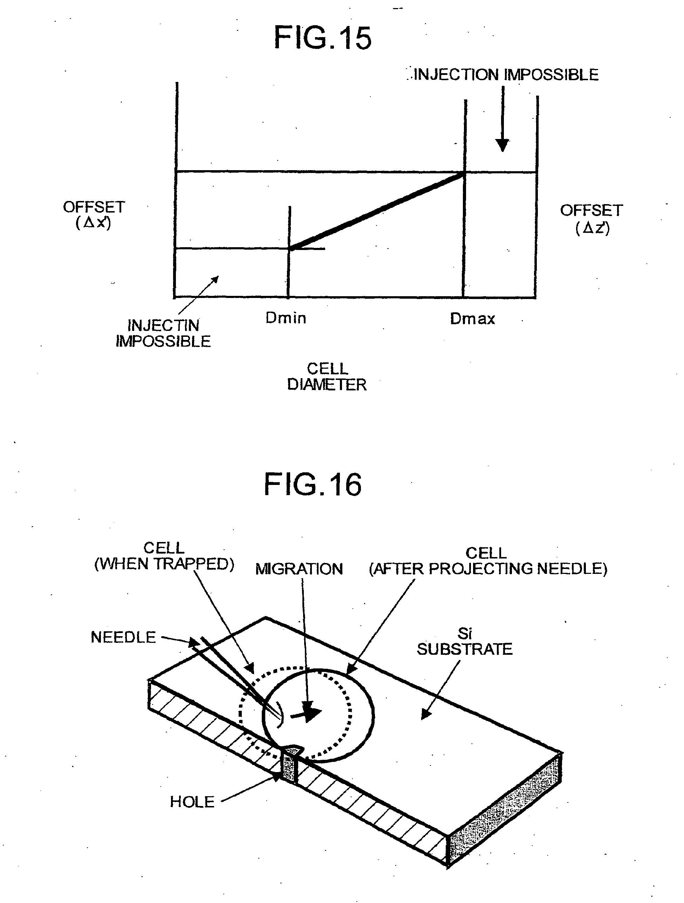

As shown in Fig. 15, when the cell is too small, injection is impossible, so that injection is suspended. Also, when the cell is too large, no injection is performed because of the cell being abnormal.

As described above, according to the fifth embodiment, the size of the cell is measured and the position of the XYZ table is adjusted based on the size of the cell, so that injection can be performed accurately even when the size of the cell fluctuates.

A microinjection apparatus according to a sixth embodiment of the present invention is configured so as to prevent migration of cells when injection is performed. First, the migration of cell when injection is performed is explained. Fig. 16 is a schematic for explaining the migration of a cell while the injection is performed.

The microinjection apparatus traps cells by suction of a culture broth from below through holes formed in the Si substrate. If the holes are 1/3 times the size of the cells, the cells pass through the hole. On the other hand, if the holes are small, problems occur that the cells do not get trapped easily, the cells do not firmly fix in the holes, and the cells move, so that the needle cannot penetrate the cell membrane, as shown in Fig. 16.

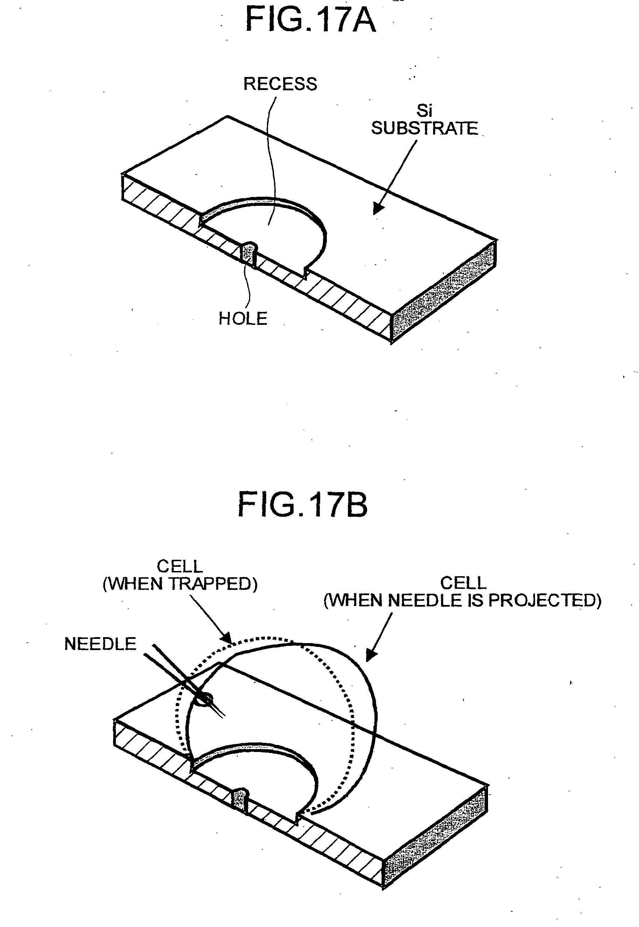

Accordingly, as shown in Fig. 17A, the diameters of the holes are made smaller (about 1/10 times the cell diameter) so that the cell does not pass through the holes, and a recess having a diameter of about 80% of the cell diameter is formed around each of the holes. Because the cells fit in these recesses, migration of the cell can be prevented.

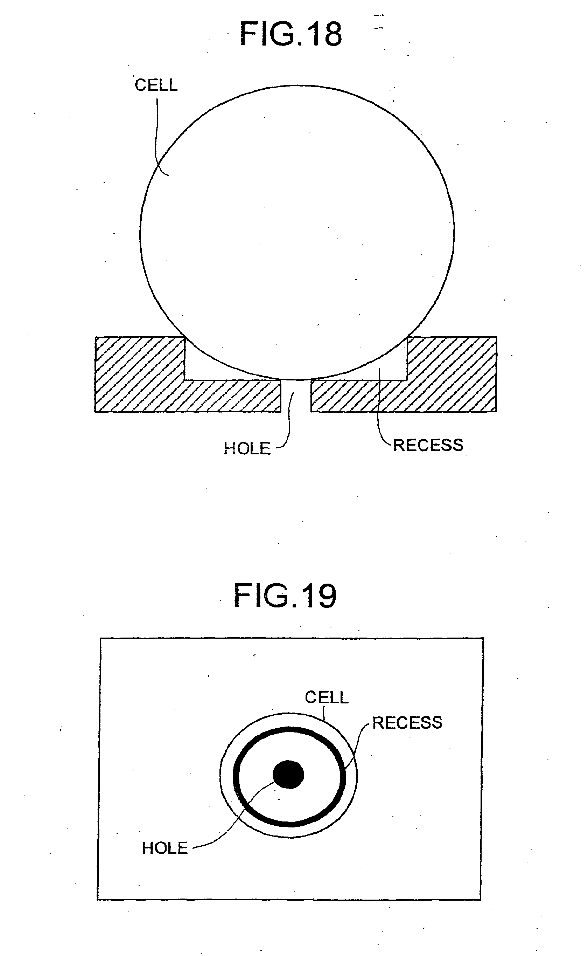

Further, Fig. 17B is a perspective of a cell trapped in the recess shown in Fig. 17A. The cell deforms more or less when the injection needle cell is injected in the cell, however, the cell does not migrate because it is fit in the recess, so that injection can be performed easily. Fig. 18 is a cross-sectional view of the substrate in a situation where a cell is trapped in the recess. When the cell touches the bottom of the recess, the cell can be trapped stably.

The cells that are about ±30% larger than the diameter of the recess can be trapped in the recesses. Fig. 19 is a plan view of the situation shown in Fig. 18.

As described above, in the sixth embodiment, the diameters of the holes are made about 1/10 time the cell diameter and a recess having a diameter of about 80% of the cell diameter is formed around each of the holes, so that the cells fit in these recesses and do not move when injection is performed.

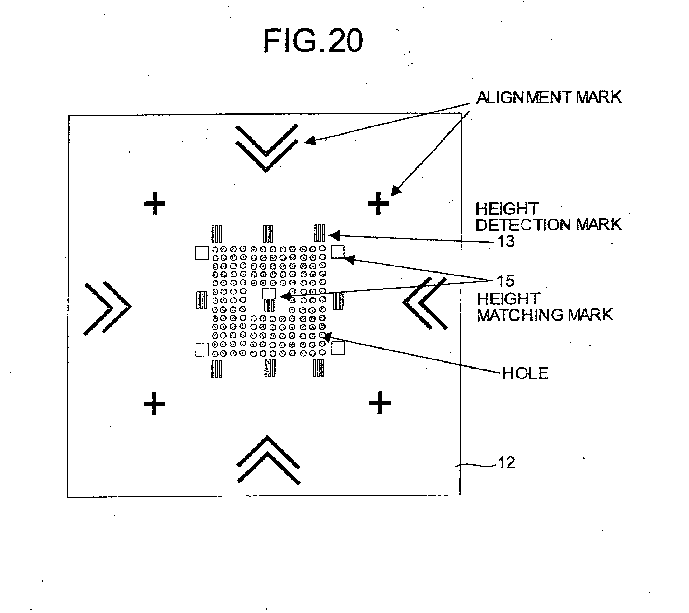

Fig. 20 is a plan view of a substrate according to a seventh embodiment of the present invention. This substrate according to the seventh embodiment is provided with both the height detection marks 13 as in the first embodiment and the height matching marks 15, as in the second embodiment.

As shown in Fig. 20, the Si chip 12 has chevron marks that indicate main directions and cross marks as alignment marks in the periphery and the height detection mark 13 and the height matching mark 13 as adjustment marks in the region where the holes are present. The center portion of the region where the holes are present deforms the most so that the height detection mark 13 and the height matching mark 15 are provided in and around this region.

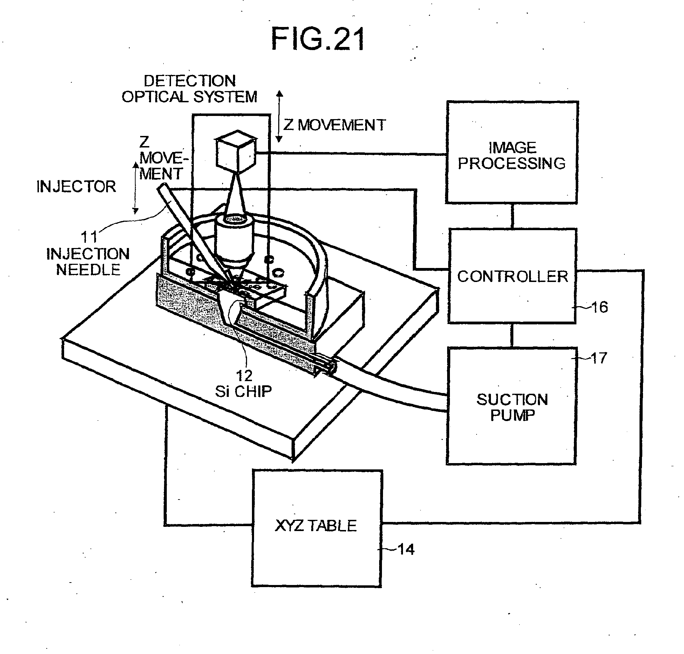

Fig. 21 is a schematic of an injection position adjustment system according to an eighth embodiment of the present invention. This injection position adjustment system can be used in any of the first to the seventh embodiments. The injection position adjustment system acquires an image using a CCD camera and measures the positions of the cell, the injection needle 11, and so on.

Based on the measured information, control of the suction amount of a suction pump 17, up and down of the XYZ table 14 and detection optical system, adjustment of the position of an injector and so on is performed. Then, after the detection position is adjusted, operations such as projection of the injection needle 11 and ejection the drug solution are performed. These operations are controlled by a controller 16.

According to the present invention, since the injection position is controlled with high precision, the present invention has an effect that the substance can be injected into the cell reliably.

Although the invention has been described with respect to a specific embodiment for a complete and clear disclosure, the appended claims are not to be thus limited but are to be construed as embodying all modifications and alternative constructions that may occur to one skilled in the art that fairly fall within the basic teaching herein set forth.

**Claims**

1. A microinjection apparatus comprising:

   a substrate having a hole for trapping a cell using suction force and injecting a substance into the cell with a needle;
   an image acquiring unit that acquires an image of a region on the substrate, the image having characteristics that change based on a deformation of the substrate;
   a calculating unit that calculates an amount of deformation of the substrate based on the image acquired; and
   a controlling unit that controls a relative position of the needle and the substrate based on the amount of deformation.

2. The microinjection apparatus according to claim 1, wherein a pattern of fine, parallel lines is provided in the region on the substrate,
   the image acquiring unit acquires an image of the pattern,
   the calculating unit calculates the amount of deformation using a change in signal intensities of the pattern in the image acquired, and calculates an amount of correction based on the amount of deformation, and
   the controlling unit controls the relative position of the needle and the substrate based the amount of correction.

3. The microinjection apparatus according to claim 1, wherein a reflecting member that reflects light is provided in the region,
   the image acquiring unit acquires a real image and a mirror image, which is a projection of a tip of the needle in the reflecting member,
   the calculating unit that calculates a distance between the tip and the substrate using the real image and the mirror image, and

   the controlling unit controls the relative position of the needle and the substrate so that the distance between the tip and the substrate is maintained at a predetermined value when the substrate and the needle move relatively in a horizontal direction.

4. The microinjection apparatus according to claim 3, the image acquiring unit includes an optical system, wherein the image acquiring unit acquires the real image and the mirror image simultaneously by shifting the optical system in a horizontal direction.

5. The microinjection apparatus according to claim 1, wherein the image acquiring unit is a charged coupled device camera.

6. The microinjection apparatus according to claim 1, wherein the substrate is made of silica.

7. A microinjection apparatus comprising:

   a substrate having a hole for trapping a cell using suction force and injecting a substance into the cell with a needle;
   a searching unit that searches a cell-free region that is a region where no cells exist on the substrate;
   a calculating unit that calculates a center of the cell-free region;
   a needle controlling unit that controls the needle so that a tip of the needle approaches the center of the cell-free region;
   a measuring unit that measures a position of the tip using an image of the tip while the needle is being controlled by the controlling unit; and
   a controlling unit that controls a relative position of the needle and the substrate based on the position of the needle measured.

8. The microinjection apparatus according to claim 7, wherein the substrate is made of silica.

9. A microinjection apparatus comprising:

   a substrate having a hole for trapping a cell using suction force and injecting a substance into the cell with a needle;
   a measuring unit that measures a size of a cell that is trapped in the hole; and
   a controlling unit that controls a relative position of the needle and the substrate based on the size of the cell measured.

10. The microinjection apparatus according to claim 9, wherein the substrate is made of silica.

11. A microinjection method including trapping a cell in a hole provided in a substrate with suction force and injecting a substance into the cell with a needle, comprising:

acquiring an image of a region on the substrate, the image having characteristics that change based on a deformation of the substrate; calculating an amount of deformation of the substrate based on the image acquired; and controlling a relative position of the needle and the substrate based on the amount of deformation.

**12.** The microinjection method according to claim 11, wherein the substrate is made of silica.

**13.** A microinjection method including trapping a cell in a hole provided in a substrate with suction force and injecting a substance into the cell with a needle, comprising:

searching a cell-free region that is a region where no cells exist on the substrate; calculating a center of the cell-free region; controlling the needle so that a tip of the needle approaches the center of the cell-free region; measuring a position of the tip using an image of the tip while the needle is being controlled; and controlling a relative position of the needle and the substrate based on the position of the needle measured.

**14.** The microinjection method according to claim 13, wherein the substrate is made of silica.

**15.** A microinjection method including trapping a cell in a hole provided in a substrate with suction force and injecting a substance into the cell with a needle, comprising:

measuring a size of a cell that is trapped in a hole; and controlling a relative position of the needle and the substrate based on the size of the cell.

**16.** The microinjection method according to claim 15, wherein the substrate is made of silica.

**17.** A microinjection apparatus comprising:

a substrate having a hole for trapping a cell using suction force and injecting a substance into the cell with a needle; a recess around each of the holes.

**18.** The microinjection apparatus according to claim 17, wherein the substrate is made of silica.

**19.** The microinjection apparatus according to claim 17, wherein a diameter of the recess is about 80% of a diameter of the cell.

# FIG.1

HEIGHT DETECTION MARK
13

12
Si CHIP
(Si SUBSTRATE)

# FIG.2

CCD CAMERA

OBJECTIVE
LENS

PETRI DISH

CELL

Si CHIP
12

14
XYZ TABLE

13
HEIGHT DETECTION MARK

SUCTION HOLE
(TO SUCTION PUMP)

# FIG.3A

HOLE    CELL

OBSERVATION LINE →

13
HEIGHT DETECTION MARK

# FIG.3B

VISIBILITY = (b-a)/(a+b)

FOCUSED TIME SIGNAL

b

b'

NON-FOCUSED TIME SIGNAL

a'

a

INTENSITY OF SIGNAL

X POSITION

# FIG.4

VISIBILITY = (b-a)/(a+b)

# FIG.5

INJECTION NEEDLE
11

Si CHIP
12

OPTIMAL DISTANCE

GLUE

14
XYZ TABLE

CENTRAL PORTION

COLLISION

HEIGHT ADJUSTMENT NECESSARY

INJECTION NEEDLE
11

Si CHIP
12

14
XYZ TABLE

MOVEMENT

PERIPHERAL PORTION

EP 1 595 941 A2

# FIG.6

CCD CAMERA

INJECTION NEEDLE
11

OBJECTIVE LENS

HEIGHT MATCHING MARK
15

Si CHIP
12

# FIG.7A

OBJECTIVE LENS

INJECTION NEEDLE
11

HEIGHT:h

# FIG.7B

$\Delta z$ MOVEMENT

INJECTION NEEDLE
11

$\Delta z = 2h$

MIRROR IMAGE

# FIG.8A

OBJECTIVE LENSE

NEEDLE TIP

h

θ  OPTIC AXIS

# FIG.8B

Δy

Δd

$\Delta y = 2 \cdot h \cdot \sin \theta$

$\therefore h = \Delta y / (2 \cdot \sin \theta)$

$\theta = \Delta d / f$

$h = \Delta y f / 2 \cdot \Delta d \ (\theta \ll 1)$

# FIG.9

# FIG.10

# FIG.11A

CELL-FREE REGION

FACE FORMED BY
TRAJECTORY OF
CENTER

CENTER

# FIG.11B

MODEMENT OF XYZ TABLE

NEEDLE CENTER LINE

CELL-FREE REGION

EP 1 595 941 A2

# FIG.12A

INJECTION NEEDLE
11

CELL

HOLE

$\Delta y_C$

$\Delta x_C$

# FIG.12B

CORRECTION OF XYZ TABLE

$-\Delta y_C + \varepsilon$

HOLE

$-\Delta x_C$

19

# FIG.13

APPROXIMATION CIRCLE

INJECTION NEEDLE
11

CELL

CENTER OF CELL

HOLE

# FIG.14

PLAN VIEW

CROSS-SECTIONAL VIEW

# FIG.15

INJECTION IMPOSSIBLE

OFFSET
($\Delta x$)

OFFSET
($\Delta z$)

INJECTIN
IMPOSSIBLE

Dmin

Dmax

CELL
DIAMETER

# FIG.16

CELL
(WHEN TRAPPED)

MIGRATION

CELL
(AFTER PROJECTING NEEDLE)

NEEDLE

Si
SUBSTRATE

HOLE

# FIG.17A

RECESS

Si
SUBSTRATE

HOLE

# FIG.17B

CELL
(WHEN TRAPPED)

CELL
(WHEN NEEDLE IS PROJECTED)

NEEDLE

# FIG.18

CELL

HOLE

RECESS

# FIG.19

CELL

RECESS

HOLE

# FIG.20

ALIGNMENT MARK

HEIGHT
DETECTION MARK
13

15

HEIGHT
MATCHING MARK

HOLE

12

# FIG.21

DETECTION
OPTICAL SYSTEM

Z MOVEMENT

Z
MOVE-
MENT

INJECTOR

11
INJECTION
NEEDLE

12
Si CHIP

IMAGE
PROCESSING

CONTROLLER

~16

~17

SUCTION
PUMP

XYZ TABLE

~14

# FIG.22

INJECTIN NEEDLE
(CAPILLARY)
11

CELL

Si CHIP
12

CULTURE FLUID
(MEDIUM)

SUCTION

BSEMENT OF;
PETRI DISH

PETRI DISH

# FIG.23

FLUCTUATION OF
NEEDLE POSITION

INJECTION NEEDLE

FLUCTUATION OF
CELL POSITION

$\Delta z$

$\Delta x$

$\Delta y$

$\theta$

FLUCTUATION OF
SUBSTRATE
(ROTATION OF
SUBSTRATE)

# FIG.24

INJECTION
NEEDLE
11

INJECTION IMPOSSIBLE

CELL(LARGE)

DEPRESSION

HOLE

INJECTION
NEEDLE
11

INJECTION IMPOSSIBLE

ADJUSTMENT
NECESSARY

CELL (SMALL)

DEPRESSION

# FIG.25A

# FIG.25B

# FIG.26A

MAXIMUM
DEFORMATION

AMOUNT OF
ADJUSTMENT OF SUCTION

# FIG.26B

100%
ADSORPTION

50%
ADSORPTION

AMOUNT OF
DEFORMATION

NO CELL
ADSORPTION

START

0    AMOUNT OF SUCTION